# EUROPEAN PATENT SPECIFICATION

(11) **EP 2 074 990 B1**
(45) Date of publication and mention of the grant of the patent: **10.08.2016**
(21) Application number: 08172947.7
(22) Date of filing: 25.12.2008
(51) Int. Cl.: A61K 9/20, A61K 31/19, A61K 45/06

(54) **Controlled release flurbiprofen and muscle relaxant combinations**
Flurbiprofen mit kontrollierter Freisetzung und Muskelentspannungskombinationen
Flurbiprofène à libération prolongée et combinaisons pour le relâchement musculaire

(30) Priority: 26.12.2007 TR 200708925
(43) Date of publication of application: 01.07.2009
(73) Proprietor: Sanovel Ilaç Sanayi Ve Ticaret Anonim Sirketi, 34460 Sariyer/Istanbul (TR)
(72) Inventor: Cifter, Ümit, 34398, Istanbul (TR); Türkyilmaz, Ali, 34398, Istanbul (TR); Turp, Hasan Ali, 34398, Istanbul (TR)
(74) Representative: Sevinç, Erkan

(56) References cited:
- WO-A-86/03681
- WO-A-98/52545
- WO-A-2007/072503

## Description

### Technical Aspect

This invention is a novel controlled release (CR) flurbiprofen and muscle relaxant combinations for oral administration with anti-inflammatory, analgesic, myorelaxant activity and methods of its manufacture. The pharmaceutical composition of the present invention is administered orally in tablet, multilayer tablet, multicoated tablet and capsule form.

More particularly, this present invention relates to a pharmaceutical composition comprising a controlled release phase I comprising an effective amount of flurbiprofen or a pharmaceutically acceptable salt thereof and one or more pharmaceutically acceptable excipient(s); and a controlled release or immediate release phase II comprising an effective amount of thiocolchicoside or tizanidine or a salt thereof, and one or more pharmaceutically acceptable excipient(s).

### Background of the Invention

Flurbiprofen is a propionic acid derivative, is a known NSAID (non-steroidal anti-inflammatory drug) with analgesic and anti-inflammatory activity. Its chemical structure is shown in the Formula 1.

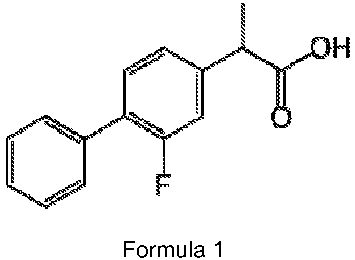

The chemical name of flurbiprofen is 2-Fluoro-α-methyl-[1,1'-biphenyl]-4-acetic acid. It is used in musculoskeletal and joint disorders such as ankylosing spondylitis, osteoarthritis and rheumatoid arthritis, in soft-tissue disorders such as sprains and strains, for postoperative pain and mild to moderate pain including dysmenorrhoea and migraine. Flurbiprofen is also used as lozenges in the symptomatic relief of sore throat. Flurbiprofen sodium is used in eye drops to inhibit intra-operative miosis and to control postoperative inflammation of the anterior segment of the eye. Flurbiprofen axetil has been given in some countries by intravenous injection for severe pain.

For pain and inflammation, flurbiprofen is given in usual doses of 150 mg to 200 mg daily by mouth in divided doses, increased to 300 mg daily in acute or severe conditions if necessary (Sean C Sweetman, Martindale The Complete Drug Reference, thirty-fifth edition 2007, Vol. 1, pages 52 to 53).

Muscle relaxants are used in the management of musculoskeletal and neuromuscular disorders. There are two main types; centrally acting relaxants and directly acting relaxants.

Centrally acting relaxants generally have a selective action on the central nervous system (CNS) and are principally used for relieving painful muscle spasms or spasticity occurring in musculoskeletal and neuromuscular disorders. Their mechanism of action may be due to their CNS-depressant activity.

Tizanidine is an example for this group of muscle relaxants. Its chemical structure is shown in Formula 2.

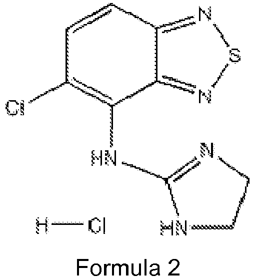

Tizanidine is a α₂-adrenergic agonist and acts mainly at spinal and supraspinal levels to inhibit excitatory interneurones. It is used for the symptomatic relief of spasticity associated with multiple sclerosis or with spinal cord injury or disease. It is also used in the symptomatic treatment of painful muscle spasm associated with musculoskeletal conditions (Sean C Sweetman, Martindale The Complete Drug Reference, thirty-fifth edition 2007, Vol. 1, page 1727).

Muscle relaxants also reduce muscle tone and are used in therapy for the treatment of muscle spasm and contractures.

Muscle spasm is one of the main factors responsible for chronic pain; it characterises several pathologies of the locomotor apparatus as well as inflammatory-rheumatic and degenerative orthopaedic pathologies. When it affects joints, they cause not only pain, but also rigidity, which reduces joint mobility and flexibility in the affected part. Muscle contractures also characterize several pathologies of the locomotor apparatus and are one of the main factors responsible for the persistence of the pain associated to these pathologies.

For these reasons, the study of molecules endowed with muscle relaxant and antispasmodic properties still raises remarkable interest from the clinical point of view.

As it is known, colchicine is a pseudoalcaloid that has been widely used for a long time in therapy for the treatment of gout. The use of 3- demethyl- thiocolchicine glucoside, known as thiocolchicoside, is also widespread in therapy for treating contractures and inflammatory conditions that affect the muscular system (Ortopedia e Traumatologia Oggi XII, n. 4, 1992).

Thiocolchicoside has been claimed to possess GABA-mimetic and glycinergic actions. In other words we can say that thiocolchicoside is a gamma-aminobutiric acid receptor agonist. Its chemical structure is shown in Formula 3.

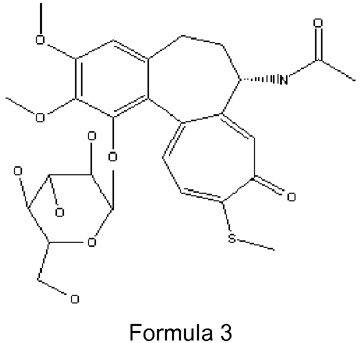

It has recently been shown that thiocoichicoside's activity can be ascribed to its ability to interact with the strychnine-sensitive glycine receptors and therefore that compounds endowed with glycino-mimetic activity can be used in the rheumatologic- orthopedic field for their muscle relaxant properties.

The usual initial dose is 16 mg daily by mouth. It has also been given intramuscularly, in doses up to 8 mg daily, or applied as cream or ointment (Sean C Sweetman, Martindale The Complete Drug Reference, thirty-fifth edition 2007, Vol. 1, page 1738).

Tizanidine and/or thiocolchicoside are known muscle relaxant agents used in the treatment of painful muscle spasms or spasticity occurring in musculoskeletal and neuromuscular disorders and for treating contractures and inflammatory conditions that affect the muscular system.

Muscle relaxants have been evaluated alone or in combination with conventional analgesics for the treatment of pain. But controlled release formulation of flurbiprofen has not been previously combined with controlled release or immediate release formulations of thiocolchicoside or tizanidine in a pharmaceutical composition for the treatment of inflammatory, pain and musculoskeletal diseases.

European patent EP 1 052 995 B1 (SANOFI-SYNTHELABO) 05.02.1998, concerns a pharmaceutical composition for nasal administration of thiocolchicoside, with immediate or sustained release.

PCT application WO 2007/016676 A1 (Teva Pharmaceutical Industries Ltd) 01.08.2005, relates to methods of treating spasticity in patient having a neurological disease comprising administering to a patient in need of such treatment a tizanidine formulation providing a tizanidine blood concentration of at least about 900 pg/ml for about five hours, wherein the formulation is administered prior to bedtime. The tizanidine formulation may be a controlled release formulation, a sublingual formulation, buccal formulation, or a high dose immediate release formulation. The controlled release formulation may be in the form of a tablet, capsule, lozenge, troche, pastille, pill, drop, gel, viscous liquid, or spray. The sublingual formulation may also be in the form of a tablet, capsule, lozenge, troche, pastille, pill, drop, gel, viscous liquid, or spray.

PCT application WO 2005/046648 A1 (Glenmark Pharmaceuticals Ltd.) 12.11.2003, discloses an extended release pharmaceutical formulation containing at least an alpha-2 adrenergic agonist, such as tizanidine, for the treatment and prevention of spasticity in a subject, e.g., painful inflammatory conditions associated with skeletal muscle spasms.

It is well known that drugs used in the same therapeutic area or even for treating the same indication cannot always be combined *a priori* with the expectation of at least additive therapeutic effects. The scientific literature is full of examples wherein compounds of different classes, which are used to treat the same indications, cannot be combined into safe and efficacious dosage forms thereby resulting in incompatible drug combinations. The reasons for this unexpected lack of compatibility are varied; however, it is often found that the incompatible drug combinations result in increased side effects, undesirable drug interactions or new side effects. More specifically, in the area of analgesia there are drug combinations that are contraindicated for some or all of these very same reasons.

The main challenges of combining two or more molecules in the same pharmaceutical form are (a) to ensure the chemico-physical compatibility between the different active ingredients and/or between the active ingredients and the excipients used; and (b) to ensure the therapeutical compatibility between the two active ingredients regarding their pharmacokinetic and/or pharmaceutical properties in order that the posology of the combined composition allows to obtain safe and efficient plasma levels of both pharmacological agents.

Conventional analgesic and myorelaxant therapy generally involves administration of a pharmaceutical composition containing one or more different analgesic and muscle relaxant drugs. However, not all analgesic and muscle relaxant drug combinations are more suitable, in terms of safety or reduced side effects; greater convenience; or have higher levels of patient compliance due to the simplified dosage schedule, as compared to those of immediate-release formulation. Accordingly, there remains a need to develop a controlled release pharmaceutical formulation of flurbiprofen with controlled release or immediate release muscle relaxants selected from the group comprising tizanidine and thiocolchicoside that provides once daily dosing for effective management of pain, spasticity, inflammatory symptoms and painful muscle spasms, an improved side effect profile and increased patient compliance.

### Detailed Description of the Invention

### List of Figures:

**Figure 1** shows an example of bilayer tablet formulation of the invention which (a) represents phase I comprising a controlled release flurbiprofen and (b) represents phase II comprising controlled release or immediate release thiocolchicoside or tizanidine.
**Figure 2** shows a multilayer tablet and the example of barrier layer which (c) represents barrier layer.
**Figure 3** shows an example of controlled release formulation of the invention which (e) represents the core comprising the slow released drug and (d) represents the coating layer comprising the drug which is released immediately.
**Figure 4** shows an example of controlled release formulation of the invention which (h) represents a core comprising the drug and pharmaceutically acceptable excipients and (f) represents a polymer coating layer giving slow release of the drug from this core (g) a coating layer comprising the drug which is released immediately.

Controlled-release pharmaceutical products are formulated to release the drug's active ingredient gradually and predictably over a 12-hour to 24-hour period. These formulations potentially provide greater effectiveness in the treatment of chronic conditions through more consistent delivery of the medication; reduced side effects; greater convenience; and higher levels of patient compliance due to a simplified dosage schedule, compared with those of immediate-release drugs.

This invention is a novel controlled release flurbiprofen and muscle relaxant combinations for oral administration with anti-inflammatory, analgesic, myorelaxant activity and methods of its manufacture. More particularly, the pharmaceutical composition of the present invention is administered orally in tablet, multilayer tablet, multicoated tablet and capsule form.

Another object of the present invention is to form a capsule comprising at least one controlled release tablet, granulate, pellet and mixture thereof.

Novel pharmaceutical composition in the form of a tablet or a capsule administered orally may provide a significant advance in the available treatments. Such combination therapy may also provide therapeutic improvements owing to the potential synergistic effect provided by the combination.

One of the advantages of controlled release formulation for the outpatient is reduced dosage regimens convenience and, more importantly, better assurance of compliance. For example, the reduction of a dose regimen from four times a day to three times a day allows the patient to take the prescribed drug during waking hours. Reduction of a dose regimen to twice a day allows the patient to take the prescribed drug in the morning and in the evening, which provides greater convenience; e.g., the patient is not required to carry an additional one when away from home. Of course, the most convenient dosage form is a once daily dose regimen. This also reduces the risk of the omitted tablets.

The other advantageous of controlled release formulation is to make the plasma concentration level stable by maintaining the drug in the blood stream for a longer time period. In this manner, the formulation is designed as controlled release to prevent the changes in drug plasma concentration levels and can be administered only once or twice a day for drugs that would otherwise have to be taken more frequently to maintain required blood levels. Also controlled release oral dosage forms may help to treat with reduced dosage regimens. Using lower dosages of active ingredients provide greater convenience and reduced side effects and toxicity.

Another aspect of the present invention is to provide an orally administrable controlled release pharmaceutical dosage form for the patients in need of therapeutic relief from spasticity associated with, for example, degenerative vertebra diseases as torticollis, dorsalgy, lombalgy, disk hernia, neurologic and traumatic disorders; painful muscle spasms associated with static and functional disorders of vertebra or occurred in post-operations of osteoarthritis; pain and inflammatory symptoms associated with tissue trauma. When this pharmaceutical formulation is administered once daily, it provides such therapeutic relief by releasing the active drug substance in such a manner that requisite blood levels are maintained for a time period sufficient to justify once a day dosing and thus ensure patient compliance while reducing potential side effects.

As mentioned above, this invention comprising a first phase comprising an effective amount of flurbiprofen or a pharmaceutically acceptable salt thereof and either pharmaceutically acceptable excipients; and a second phase comprising an effective amount of muscle relaxants selected from the group consisting of an α-2 adrenergic receptor agonist and a gamma-aminobutiric acid receptor agonist, and pharmaceutically acceptable excipients.

In preferred embodiments, α-2 adrenergic receptor agonists suitable for use in the context of the present invention are selected from the group comprising tizanidine, clonidine, brimonidine, apraclonidine, guanfacine, guanabenz, mivazerol, dexmedetomidine or a pharmaceutically acceptable salt thereof. Preferably, α-2 adrenergic receptor agonist is tizanidine or a pharmaceutically acceptable salt thereof.

Gamma-aminobutiric acid receptor agonists suitable for use in the context of the present invention are selected from the group comprising thiocolchicoside, baclofen and musimol or a pharmaceutically acceptable salt thereof. Preferably, the gamma-aminobutiric acid receptor agonist is a thiocolchicoside or a pharmaceutically acceptable salt thereof.

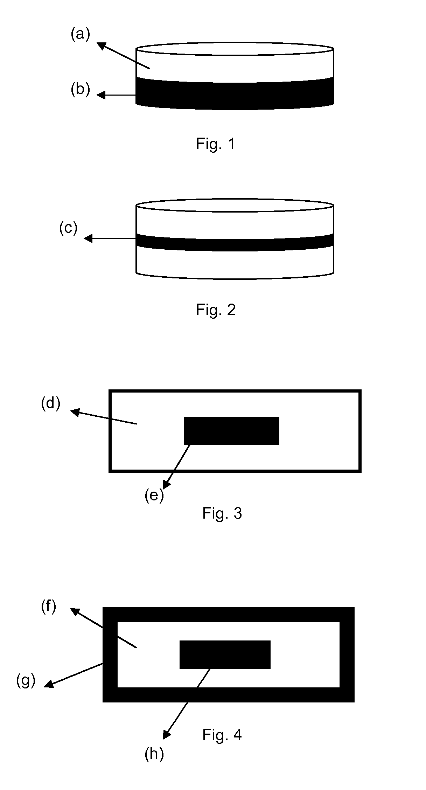

The present invention relates to a pharmaceutical composition comprising a controlled release phase I comprising an effective amount of flurbiprofen or a pharmaceutically acceptable salt thereof and either pharmaceutically acceptable excipients; and a controlled release or immediate release phase II comprising an effective amount of thiocolchicoside or tizanidine or a salt thereof, and pharmaceutically acceptable excipients. *(Figure 1)*

The present invention concern the use of pharmaceutical composition comprising controlled release flurbiprofen in combination with controlled release or immediate release tizanidine or thiocolchicoside for the manufacture of a medicament for the treatment of painful muscle spasms associated with static and functional disorders of vertebra or occurred in post-operations of osteoarthritis, pain and inflammatory symptoms associated with tissue trauma, degenerative vertebra diseases as torticollis, dorsalgy, lombalgy, disk hernia, neurologic and traumatic disorders associated with spasticity.

According to the main challenges mentioned above, the pharmaceutical composition comprising controlled release flurbiprofen in combination with tizanidine or thiocolchicoside have an additive analgesic effect in relief of postoperative pain and provide greater analgesia with the results in a lower incidence of side effects according to *priori.* These pharmaceutical combinations may be administered orally, parenterally, ocularly, nasally, buccally, sublingually and topically.

The pharmaceutical compositions of the invention include tablets, multilayer tablets, multicoated tablets and capsules which can be made in accordance with methods that are standard in the art.

Drug combinations and controlled release drug formulations will typically be prepared in admixture with pharmaceutical acceptable excipients. Suitable excipients include, but are not limited to: water; salt solutions; alcohols; gum arabic; vegetable oils; benzyl alcohol; polyethylene glycols; gelatin; carbohydrates such as lactose, amylose or starch; magnesium stearate; talc; silicic acid; paraffin; perfume oil; fatty acid esters; hydroxypropylmethylcellulose; polyvinyl pyrrolidone; etc. The pharmaceutical preparations can be sterilized and, if desired, mixed with auxiliary agents such as: lubricants, binding agents, fillers, preservatives, disintegrants, stabilizers, wetting agents, emulsifiers, salts, buffers, coloring agents, flavoring agents, coating agents, plasticizers, aromatic substances or sweeteners. Examples of oral dosage forms include tablets, multilayer tablets (coated or uncoated), multicoated tablets, capsules, hard or soft gelatin capsules, pellets, powders, granules, colloidal dispersions, dispersions, sterile solutions or suspensions and emulsions and the like.

Preferably, the combination of a controlled release flurbiprofen with tizanidine or thiocolchicoside will be in the form of a multilayer tablet. According to the present invention it may be produced by any standard tabletting technique, e.g. by wet granulation, dry granulation or direct compression.

As mentioned above, this invention is comprising a combination of controlled release flurbiprofen with tizanidine or thiocolchicoside or pharmaceutically acceptable salt thereof comprising an effective amount of fillers, polymers, binding agents, disintegrants, diluents and lubricants or their mixtures. Said invention describes a pharmaceutical combination comprising an effective amount of polymers selected from the group comprising hydroxypropylmethylcellulose (HPMC), hydroxypropylmethylcellulose phthalate (HPMCP), hydroxypropylcellulose (HPC), hydroxyethylcellulose (HEC), ethylcellulose (EC), polymethacrylates, methylcellulose (MC), sodium carboxymethylcellulose and cellulose acetate butyrate or a mixture thereof; fillers selected from the group comprising starch, lactose, microcrystalline cellulose, carboxy cellulose sodium, sucrose; an effective amount of binding agents selected from the group comprising povidone, hydroxypropylcellulose, hydroxypropylmethylcellulose, ethylcellulose, starch, gelatin; an effective amount of lubricants selected from the group comprising collodial anhydrous silica, magnesium stearate, talc, sodium stearil fumarate; an effective amount of disintegrants selected from the group comprising microcrystalline cellulose, sodium starch glycollate, croscarmellose sodium, crospovidone, starch and their mixtures; an effective amount of coating agents selected from the group comprising ethyl cellulose, polymethacrilates, triethyl citrate; an effective amount of plasticizer selected from the group comprising diethyl phthalate (DEP), triethyl citrate.

As mentioned above, this invention is comprising the active ingredients, flurbiprofen or a pharmaceutically acceptable salt thereof in combination with tizanidine wherein the flurbiprofen is present in an amount of between 50 and 500 mg and the tizanidine is present in an amount of between 2 and 36 mg, preferred embodiments of the flurbiprofen is present in an amount of between 100 and 300 mg and the tizanidine is present in an amount of between 6 and 24 mg.

This invention is further defined by reference to the following examples. Although the examples are not intended to limit the scope of the present invention, it should be considered in the light of the description detailed above.

### Example 1

### Controlled release flurbiprofen granules:

Flurbiprofen granules are granulated with high shear granulator or they are obtained from extruder to become pellets by spheronizer and after dried by fluid bed drier or dried in oven, they are sieved and pressed or matrix tablets are pressed by direct compression.

| Content | % amount (w/w) |
|---|---|
| Flurbiprofen | 20 - 70 |
| Collidon^{®} SR | 20 - 80 |
| Colloidal silicon dioxide | 0.1 - 10 |
| Magnesium stearate | 0.1 - 10 |

### Controlled release tizanidine granules:

Tizanidine granules are granulated with high shear granulator or they are obtained from extruder to become pellets by spheronizer and after dried by fluid bed drier or dried in oven, they are sieved and pressed.

| Content | % amount (w/w) |
|---|---|
| Tizanidine | 2 - 6 |
| Sucrose | 30 - 90 |
| Ethyl cellulose | 5 - 15 |
| Methacrylic acid - Methyl methacrylate copolymer | 3 - 15 |
| Hydroxypropylmethylcellulose phthalate (HPMCP) | 0,5 - 10 |
| Triethyl citrate | 0,1 - 5 |
| Talc | 0,1 - 10 |

The solid dosage form mentioned above is a bilayer tablet having the flurbiprofen granules in phase I and tizanidine granules phase II. These granules are compressed by two layered tablet press machine to obtain bilayer tablet forms and these bilayer tablets preferably covered by a coating material including conventional coating polymers like Opadry^{®}.

### Example 2

### Controlled release flurbiprofen granules:

Flurbiprofen granules are granulated with high shear granulator or they are obtained from extruder to become pellets by spheronizer and after dried by fluid bed drier or dried in oven, they are sieved and pressed or matrix tablets are pressed by direct compression.

| Content | mg amount (w/w) |
|---|---|
| Flurbiprofen | 200.00 |
| Collidon^{®} SR | 250.00 |
| Colloidal silicon dioxide | 4.00 |
| Magnesium stearate | 4.00 |
| Total : | 485.00 mg |

### Controlled release tizanidine granules:

Tizanidine granules are granulated with high shear granulator or they are obtained from extruder to become pellets by spheronizer and after dried by fluid bed drier or dried in oven, they are sieved and pressed.

| Content | mg amount (w/w) |
|---|---|
| Tizanidine HCl (equivalent to 6 mg Tizanidin) | 6.864 |
| Sucrose | 114.636 |
| Ethyl cellulose | 15.000 |
| Methacrylic acid - Methyl methacrylate copolymer | 9.500 |
| Hydroxypropylmethylcellulose phthalate (HPMCP) | 2.200 |
| Triethyl citrate | 0.300 |
| Talc | 1.500 |
| Total : | 150.00 mg |

The solid dosage form mentioned above is a bilayer tablet having the flurbiprofen granules in phase I and tizanidine granules in phase II. These granules are compressed by two layered tablet press machine to obtain bilayer tablet forms and these bilayer tablets preferably covered by a coating material including conventional coating polymers like Opadry^{®}.

As mentioned above, this invention comprising active ingredient, flurbiprofen or a pharmaceutically acceptable salt thereof in combination with thiocolchicoside wherein the flurbiprofen is present in an amount of between 50 and 500 mg and the thiocolchicoside is present in an amount of between 2 and 20 mg, preferred embodiments of the flurbiprofen is present in an amount of between 100 and 300 mg and the thiocolchicoside is present in an amount of between 4 and 16 mg.

This invention is further defined by reference to the following examples. Although the examples are not intended to limit the scope of the present invention, it should be considered in the light of the description detailed above.

### Example 3

### Controlled release flurbiprofen granules:

Flurbiprofen granules are granulated with high shear granulator or they are obtained from extruder to become pellets by spheronizer and after dried by fluid bed drier or dried in oven, they are sieved and pressed or matrix tablets are pressed by direct compression.

| Content | %amount (w/w) |
|---|---|
| Flurbiprofen | 20 - 70 |
| Collidon^{®} SR | 20 - 90 |
| Colloidal silicon dioxide | 0.1 - 10 |
| Magnesium stearate | 0.1 - 10 |

### Controlled release thiocolchicoside granules:

Thiocolchicoside granules are granulated with high shear granulator or they are obtained from extruder to become pellets by spheronizer and after dried by fluid bed drier or dried in oven, they are sieved and pressed.

| Content | %amount (w/w) |
|---|---|
| Thiocolchicoside | 5 - 20 |
| Lactose monohydrate | 25 - 85 |
| Microcrystalline cellulose | 10 - 35 |
| Hydroxypropylmethylcellulose | 5 - 20 |
| Colloidal cilicon dioxide | 0,5 - 10 |
| Magnesium stearate | 0,5 - 10 |

The solid dosage form mentioned above is a bilayer tablet having the flurbiprofen granules in phase I and thiocolchicoside granules in phase II. These granules are compressed by two layered tablet press machine to obtain bilayer tablet forms and these bilayer tablets preferably covered by a coating material including conventional coating polymers like Opadry^{®}.

Another object of the present invention is to form a multilayer tablet comprising at least one controlled release phase having the flurbiprofen in one phase and tizanidine or thiocolchicoside in another phase.

It is know in the prior art that the surface area of the pharmaceutical dosage forms is one of the most important feature for its dissolution. The bioavailabilty of the drug can be more efficient accordingly. In this present invention, another preferred embodiment is using another phase such as a barrier layer in the middle of the multilayer tablet in order to obtain a separation between both phases easily, without damaging the surface areas *(Figure 2)*. When this multilayer tablet contacts with the dissolution media they separate and act like two independent tablets. The surface area of each tablet is evaluated independently in bioavailabilty; as a result of this, the bioavailability of the tablets enhances as compared to the other multilayer tablet formulations because of the separation by the help of barrier layer.

The novel pharmaceutical composition in this present invention is administered orally in the form of a tablet, multilayer tablet, multicoated tablet and capsule. This formulation can be prescribed once-α-day or twice-α-day to a patient in need thereof to deliver the drug over, approximately in 24 hours period, with diminished incidence and decreased intensity of one or more common side effects.

In the other preferred embodiment, the pharmaceutical composition is in the form of a multicoated tablet comprising:
a core comprising the slow released drug and pharmaceutically acceptable excipients *(Figure 3(e))*,
a coating layer comprising the drug which is released immediately *(Figure 3(d))*.

The pharmaceutical composition is in the form of a multicoated tablet comprising:
a core comprising the drug and pharmaceutically acceptable excipients *(**Figure 4(h)**)*,
a polymer coating layer giving slow release of the drug from this core *(Figure 4(f))*,
a coating layer comprising the drug which is released immediately *(Figure 4(g))*.

Another object of the present invention is to form a multicoated tablet comprising a core comprising the controlled relase pellets with immediate release granules and pharmaceutically acceptable excipients, preferably with a coating layer.

This invention is further defined by reference to the following examples. Although the examples are not intended to limit the scope of the present invention, it should be considered in the light of the description detailed above.

### Example 4

### Controlled release flurbiprofen granules:

Flurbiprofen granules are granulated with high shear granulator or they are obtained from extruder to become pellets by spheronizer and after dried by fluid bed drier or dried in oven, they are sieved and pressed or matrix tablets are pressed by direct compression.

| Content | % amount (w/w) |
|---|---|
| Flurbiprofen | 20 - 70 |
| Collidon^{®} SR | 20 - 80 |
| Colloidal silicon dioxide | 0.1 - 10 |
| Magnesium stearate | 0.1 - 10 |

### Coating phase includind tizanidine granules:

| Content | %amount (w/w) |
|---|---|
| Tizanidine HCl (equivalent to 4 mg Tizanidine) | 10 - 50 |
| Hydroxypropyl methylcellulose (3Cp) | 30 - 90 |
| Poliethylen glycol (PEG 8000) | 1 - 10 |
| Talc | 1 - 20 |

Water is used for the preparation of film coating mixture.

Tizanidine and HPMC are dissolved in water and PEG and than talc is added to this mixture and it is mixed until having a homogenous mixture. The pressed controlled release flurbiprofen tablets which are mentioned in first part are coated with the coating suspension described in second part of the formulaton. These coated tablets, preferably are coated by a coating material including conventional coating polymers like Opadry^{®}.

## Claims

1. A pharmaceutical composition comprising: **(1)** a controlled release phase I comprising an effective amount of flurbiprofen or a pharmaceutically acceptable salt thereof and a pharmaceutically acceptable excipient; and **(2)** a controlled release or immediate release phase II comprising an effective amount of tizanidine or thiocolchicoside or a salt thereof, and a pharmaceutically acceptable excipient.

2. The pharmaceutical composition of claim 1, wherein the flurbiprofen or a pharmaceutically acceptable salt thereof is present in an amount of between 50 and 500 mg and the tizanidine or a pharmaceutically acceptable salt thereof is present in an amount of between 2 and 36 mg.

3. The pharmaceutical composition of claim 2, wherein the flurbiprofen or a pharmaceutically acceptable salt thereof is present in an amount of between 100 and 300 mg and the tizanidine or a pharmaceutically acceptable salt thereof is present in an amount of between 6 and 24 mg.

4. The pharmaceutical composition of claim 1, wherein the flurbiprofen or a pharmaceutically acceptable salt thereof is present in an amount of between 50 and 500 mg and the thiocolchicoside or a pharmaceutically acceptable salt thereof is present in an amount of between 2 and 20 mg.

5. The pharmaceutical composition of claim 4, wherein the flurbiprofen or a pharmaceutically acceptable salt thereof is present in an amount of between 100 and 300 mg and the thiocolchicoside or a pharmaceutically acceptable salt thereof is present in an amount of between 4 and 16 mg.

6. The pharmaceutical composition of any of the preceding claims, wherein said pharmaceutical composition is administered orally.

7. The pharmaceutical composition of Claim 6, wherein said pharmaceutical composition is formulated as capsules, tablets, multilayer tablets, multicoated tablets.

8. The pharmaceutical composition according to claim 7, in the form of a capsule comprising at least one controlled release tablet, granulate or pellet and mixture thereof.

9. The pharmaceutical composition according to claim 7, in the form of a multilayer tablet comprising at least one controlled release layer.

10. The pharmaceutical composition according to claim 7, in the form of a multicoated tablet comprising:
(a) a core comprising the slow released drug and a pharmaceutically acceptable excipient,
(b) a coating layer comprising the drug which is released immediately.

11. Use of a pharmaceutical composition of any of the preceding claims, for the manufacture of a medicament for the treatment of painful muscle spasms associated with static and functional disorders of vertebra or occurred in post-operations of osteoarthritis, pain and inflammatory symptoms associated with tissue trauma, degenerative vertebra diseases as torticollis, dorsalgy, lombalgy, disk hernia, neurologic and traumatic disorders associated with spasticity.

12. The use according to claim 11, wherein the composition is to be administered orally.

13. The use according to claim 11, wherein the composition is in the form of a capsule, tablet, multilayer tablet and multicoated tablet.

14. The pharmaceutical composition of any of the claims 1-10, wherein the composition is to be administered once-α-day or twice-α-day.

## Patentansprüche

1. Pharmazeutische Zusammensetzung aufweisend:
**(1)** eine kontrollierte-Abgabe-Phase I mit einer wirksamen Menge von Flurbiprofen oder einem pharmazeutisch akzeptablen Salz davon und einem pharmazeutisch akzeptablen Träger, und **(2)** eine kontrollierte-Abgabe oder direkte-Abgabe Phase II mit einer wirksamen Menge von Tizanidin oder Thiocolchicosid oder einem Salz davon und einem pharmazeutisch akzeptablen Träger.

2. Pharmazeutische Zusammensetzung nach Anspruch 1,
wobei das Flurbiprofen oder ein pharmazeutisch akzeptables Salz davon in einer Menge von zwischen 50 und 500 mg vorhanden ist und das Tizanidin oder ein pharmazeutisch akzeptables Salz davon in einer Menge von zwischen 2 und 36 mg vorhanden ist.

3. Pharmazeutische Zusammensetzung nach Anspruch 2,
wobei das Flurbiprofen oder ein pharmazeutisch akzeptables Salz davon in einer Menge von zwischen 100 und 300 mg vorhanden ist und das Tizanidin oder ein pharmazeutisch akzeptables Salz davon in einer Menge von zwischen 6 und 24 mg vorhanden ist.

4. Pharmazeutische Zusammensetzung nach Anspruch 1,
wobei das Flurbiprofen oder ein pharmazeutisch akzeptables Salz davon in einer Menge von zwischen 50 und 500 mg vorhanden ist und das Thiocolchicosid oder ein pharmazeutisch akzeptables Salz davon in einer Menge von zwischen 2 und 20 mg vorhanden ist.

5. Pharmazeutische Zusammensetzung nach Anspruch 4,
wobei das Flurbiprofen oder ein pharmazeutisch akzeptables Salz davon in einer Menge von zwischen 100 und 300 mg vorhanden ist und das Thiocolchicosid oder ein pharmazeutisch akzeptables Salz davon in einer Menge von zwischen 4 und 16 mg vorhanden ist.

6. Pharmazeutische Zusammensetzung nach einem der voranstehenden Ansprüche,
wobei die pharmazeutische Zusammensetzung oral verabreichbar ist.

7. Pharmazeutische Zusammensetzung nach Anspruch 6,
wobei die pharmazeutische Zusammensetzung als Kapseln, Tabletten, als mehrschichtige Tabletten, als mehrfach beschichtete Tabletten gestaltet ist.

8. Pharmazeutische Zusammensetzung nach Anspruch 7,
in Form einer Kapsel, mindestens eine kontrollierte-Abgabe Tablette, Granulat oder Pellet und Mischungen hiervon aufweisend.

9. Pharmazeutische Zusammensetzung nach Anspruch 7,
in der Form einer mehrschichtigen Tablette, mindestens eine kontrollierte-Abgabe Schicht aufweisend.

10. Pharmazeutische Zusammensetzung nach Anspruch 7,
in der Form einer mehrfach beschichteten Tablette, aufweisend:
(a) ein Kern mit einem langsam abgebbaren Medikament und einem pharmazeutisch akzeptablen Träger,
(b) einer Beschichtungsschicht mit dem Medikament, welches unmittelbar abgebbar ist.

11. Verwendung einer pharmazeutischen Zusammensetzung nach einem der voranstehenden Ansprüche, zum Herstellen eines Medikaments für die Behandlung von schmerzhaftem Muskelspasmus, welcher mit statischer oder funktionaler Störung von Wirbeln verbunden ist oder nach Arthroseoperationen auftritt, von Schmerzen und Entzündungssymptomen, welche mit einem Gewebetrauma verbunden sind, von degenerativer Wirbelknochenkrankheit als Torticollis, Dorsalgie, Lumbalgie, Bandscheibenvorfall, von neurologischer und traumatischer Störung, welche mit Spasmus assoziiert ist.

12. Verwendung nach Anspruch 11,
wobei die Zusammensetzung oral verabreicht wird.

13. Verwendung nach Anspruch 11,
wobei die Zusammensetzung in Form einer Kapsel, Tablette, einer mehrschichtigen Tablette oder einer mehrschichtig beschichteten Tablette ist.

14. Pharmazeutische Zusammensetzung nach einem der Ansprüche 1 bis 10,
wobei die Zusammensetzung einmal-täglich oder zweimal-täglich verabreicht wird.

## Revendications

1. Composition pharmaceutique comportant : (1) une phase I à libération contrôlée comportant une quantité efficace de flurbiprofène ou un sel pharmaceutiquement acceptable de celui-ci et un excipient pharmaceutiquement acceptable, et (2) une phase II à libération contrôlée ou à libération immédiate comportant une quantité effective de tizanidine ou de thiocolchicoside ou un sel de celui-ci, et un excipient pharmaceutiquement acceptable.

2. Composition pharmaceutique selon la revendication 1, dans laquelle le flurbiprofène ou un sel pharmaceutiquement acceptable de celui-ci est présent en une quantité comprise entre 50 et 500 mg et la tizanidine ou un sel pharmaceutiquement acceptable de celle-ci est présent en une quantité comprise entre 2 et 36 mg.

3. Composition pharmaceutique selon la revendication 2, dans laquelle le flurbiprofène ou un sel pharmaceutiquement acceptable de celui-ci est présent en une quantité comprise entre 100 et 300 mg et la tizanidine ou un sel pharmaceutiquement acceptable de celle-ci est présent en une quantité comprise entre 6 et 24 mg.

4. Composition pharmaceutique selon la revendication 1, dans laquelle le flurbiprofène ou un sel pharmaceutiquement acceptable de celui-ci est présent en une quantité comprise entre 50 et 500 mg et le thiocolchicoside ou un sel pharmaceutiquement acceptable de celui-ci est présent en une quantité comprise entre 2 et 20 mg.

5. Composition pharmaceutique selon la revendication 4, dans laquelle le flurbiprofène ou un sel pharmaceutiquement acceptable de celui-ci est présent en une quantité comprise entre 100 et 300 mg et le thiocolchicoside ou un sel pharmaceutiquement acceptable de celui-ci est présent en une quantité comprise entre 4 et 16 mg.

6. Composition pharmaceutique selon l'une quelconque des revendications précédentes, dans laquelle ladite composition pharmaceutique est administrée par voie orale.

7. Composition pharmaceutique selon la revendication 6, dans laquelle ladite composition pharmaceutique est formulée sous forme de gélules, de comprimés, de comprimés multicouches ou de comprimés à enrobage multiple.

8. Composition pharmaceutique selon la revendication 7, se présentant sous forme de gélule comportant au moins un comprimé, un granule ou une pastille à libération contrôlée et un mélange de ceux-ci

9. Composition pharmaceutique selon la revendication 7, se présentant sous forme de comprimé multicouche comportant au moins une couche à libération contrôlée.

10. Composition pharmaceutique selon la revendication 7, se présentant sous forme de comprimé à enrobage multiple comportant :
(a) un noyau comportant le médicament à libération lente et un excipient pharmaceutiquement acceptable,
(b) une couche d'enrobage comportant le médicament qui est libéré immédiatement.

11. Utilisation d'une composition pharmaceutique selon l'une quelconque des revendications précédentes, pour la fabrication d'un médicament destiné au traitement de spasmes musculaires douloureux associés à des troubles statiques et fonctionnels de vertèbres ou ayant eu lieu lors de post-opérations d'ostéoarthrite, de douleurs et de symptômes inflammatoires associés à des traumatismes tissulaires, de maladies dégénératives des vertèbres telles qu'un torticolis, une dorsalgie, une lombalgie, une hernie discale, des troubles neurologiques et traumatiques associés à une spasticité.

12. Utilisation selon la revendication 11, dans laquelle la composition doit être administrée par voie orale.

13. Utilisation selon la revendication 11, dans laquelle la composition se présente sous forme de gélule, de comprimé, de comprimé multicouche et de comprimé à enrobage multiple.

14. Composition pharmaceutique selon l'une quelconque des revendications 1 à 10, dans laquelle la composition doit être administrée une ou deux fois par jour.
